# EUROPÄISCHE PATENTANMELDUNG

(11) **EP 3 834 723 A1**
(43) Veröffentlichungstag der Anmeldung: **16.06.2021**
(21) Anmeldenummer: 20207334.2
(22) Anmeldetag: 13.11.2020
(51) Int. Cl.: A61B 5/12, A61B 5/00

(54) **VERFAHREN ZUR ERMITTLUNG DER HÖRSCHWELLE EINER TESTPERSON**

(30) Priorität: 11.12.2019 DE 102019219385
(71) Anmelder: Sivantos Pte. Ltd., Singapore 539775 (SG)
(72) Erfinder: GIESE, Ulrich, 90765 Fürth (DE); DROSTE, Eva, 91054 Erlangen (DE)
(74) Vertreter: FDST Patentanwälte

(57) **Zusammenfassung**

Zur Ermittlung der Hörschwelle einer Testperson werden verfahrensgemäß einer Testperson mittels eines Ausgabewandlers (6) nacheinander mehrere Tonsätze, die mehrheitlich eine Mehrzahl von Geräuschen mit innerhalb eines Tonsatzes gleichbleibenden Eigenschaften enthalten, dargeboten, von der Testperson nach jeder Darbietung eines dieser Tonsätze die Angabe einer wahrgenommenen Anzahl von Geräuschen aus diesem dargebotenen Tonsatz abgefragt, in Abhängigkeit von der wahrgenommenen Anzahl von Geräuschen für die Darbietung eines nachfolgenden Tonsatzes eine Eigenschaft zumindest eines Teils der Geräusche gegenüber dem vorausgehenden Tonsatz verändert und der nachfolgende Tonsatz der Testperson dargeboten, und in Abhängigkeit von der jeweiligen wahrgenommenen Anzahl von Geräuschen in den dargebotenen Tonsätzen wenigstens ein Wert der Hörschwelle der Testperson abgeschätzt wird.

## Beschreibung

Die Erfindung betrifft ein Verfahren zur Ermittlung der Hörschwelle einer Testperson. Des Weiteren betrifft die Erfindung ein Verfahren zum Einstellen von Hörgeräteparametern eines Hörgeräts. Außerdem betrifft die Erfindung ein Hörgerätesystem .

Hörgeräte dienen im Allgemeinen zur Wiedergabe von Tonsignalen an das Gehör eines Nutzers des jeweiligen Hörgeräts. Dazu umfassen Hörgeräte zumindest einen Ausgabewandler, üblicherweise einen Lautsprecher (auch: "receiver"). In Form von sogenannten Hörhilfegeräten dienen Hörgeräte zur Versorgung von in ihrem Hörvermögen verminderten Personen mit Tonsignalen. Regelmäßig umfassen Hörhilfegeräte dazu wenigstens ein Mikrofon, einen Signalprozessor zur meist nutzerspezifischen, frequenzabhängigen Filterung, Verstärkung und/oder Dämpfung von mittels des Mikrofons erfassten Geräuschen, sowie den vorgenannten Ausgabewandler. Je nach Art der Hörminderung kann der Ausgabewandler neben dem vorstehend beschriebenen Lautsprecher auch als Knochenleitungshörer oder Cochlea-Implantat zur mechanischen bzw. elektrischen Stimulation des Gehörs des Nutzers ausgebildet sein. Neben den Hörhilfegeräten fallen aber grundsätzlich auch Kopfhörer, Headsets, "hearables" und dergleichen und den Begriff "Hörgerät".

Zur individuellen Anpassung der Signalverarbeitung im Signalprozessor wird üblicherweise ein sogenanntes Audiogramm des Nutzers erstellt. Aus diesem kann wiederum die sogenannte Hörschwelle, d. h. ab welchem Lautstärkewert der Nutzer einen Ton einer spezifischen Tonfrequenz hören kann, ausgelesen oder ermittelt werden. Herkömmlicherweise werden dem Nutzer dabei zunächst nacheinander Töne steigender oder zumindest veränderter Tonfrequenz präsentiert, wobei die Lautstärke in ihrem Wert (kontinuierlich oder schrittweise) zunimmt. Dieses Testverfahren ist allerdings vergleichsweise fehleranfällig, da der Nutzer häufig erwartet, innerhalb eines Zeitraums nach Testbeginn oder Bestätigung eines vorausgehenden Tons einen weiteren Ton veränderter Tonfrequenz präsentiert zu bekommen und kann somit "blind", also ohne den Ton tatsächlich zu hören, den Ton bestätigen. Andererseits ist dieses Testverfahren vergleichsweise einfach.

Der Erfindung liegt die Aufgabe zugrunde, eine möglichst robuste Ermittlung der Hörschwelle zu ermöglichen.

Diese Aufgabe wird erfindungsgemäß gelöst durch ein Verfahren mit den Merkmalen des Anspruchs 1. Des Weiteren wird diese Aufgabe erfindungsgemäß gelöst durch ein Computerprogrammprodukt mit den Merkmalen des Anspruchs 13 sowie durch ein Verfahren mit den Merkmalen des Anspruchs 14. Außerdem wird diese Aufgabe erfindungsgemäß gelöst durch ein Hörgerätesystem mit den Merkmalen des Anspruchs 15. Vorteilhafte und teils für sich erfinderische Ausführungsformen und Weiterentwicklungen der Erfindung sind in den Unteransprüchen und der nachfolgenden Beschreibung dargelegt.

Das erfindungsgemäße Verfahren dient zur Ermittlung der Hörschwelle einer Testperson. Als "Hörschwelle" wird dabei insbesondere der niedrigste Wert der Lautstärke (oder auch: des Pegels oder des Levels) eines akustischen Signals, insbesondere eines Geräuschs (bspw. eines Tons) verstanden, bei dem die Testperson dieses Geräusch noch wahrnehmen, also hören kann. Die Hörschwelle ist somit insbesondere frequenzabhängig.

Im Rahmen des erfindungsgemäßen Verfahrens werden einer Testperson mittels eines Ausgabewandlers nacheinander mehrere Tonsätze präsentiert (oder: dargeboten), die mehrheitlich eine Mehrzahl von Geräuschen, insbesondere mit jeweils innerhalb eines Tonsatzes gleichbleibenden, vorzugsweise aber untereinander zumindest zum Teil unterschiedlichen, Eigenschaften, enthalten. Von der Testperson wird nach jeder Darbietung eines dieser Tonsätze die Angabe einer wahrgenommenen Anzahl von Geräuschen aus diesem (d. h. insbesondere dem unmittelbar vorausgehend) dargebotenen Tonsatz abgefragt. In Abhängigkeit von der wahrgenommenen Anzahl von Geräuschen wird wenigstens für die Darbietung des nachfolgenden Tonsatzes eine Eigenschaft zumindest eines Teils der Geräusche (in diesem nachfolgenden Tonsatz) gegenüber dem vorausgehenden Tonsatz verändert. Dieser veränderte, nachfolgende Tonsatz wird dann der Testperson dargeboten. In Abhängigkeit von der jeweiligen wahrgenommenen Anzahl von Tönen in den (insbesondere mindestens zwei, vorzugsweise mehr als zwei) dargebotenen Tonsätzen wird wenigstens ein Wert - insbesondere ein einzelner, vorzugsweise frequenzspezifischer Wert und/oder ein Verlauf über mehrere Tonfrequenzen hinweg - der Hörschwelle der Testperson abgeschätzt.

Anders ausgedrückt, wird der Testperson ein Tonsatz mit mehreren (insbesondere mit untereinander unterschiedlichen aber für sich jeweils gleichbleibenden Eigenschaften) Geräuschen vorgespielt. Zu diesem Tonsatz muss die Testperson eine Angabe über die darin enthaltene, wahrgenommene Anzahl von Geräuschen machen. In Abhängigkeit von den für den ersten Tonsatz gewählten Eigenschaften der Geräusche lässt sich eventuell bereits ein erster Schätzwert für die Hörschwelle abschätzen, insbesondere dann, wenn die Testperson nicht alle im Rahmen dieses Tonsatzes dargebotenen Geräusche gehört hat (d. h. als hörbar angegeben hat). Zur Verifizierung und/oder präziseren Bestimmung des Schätzwerts der Hörschwelle wird dann wenigstens ein weiterer Tonsatz dargeboten, für dessen Geräusche zumindest teilweise die Eigenschaften verändert werden. Insbesondere für den Fall, dass in dem ersten Tonsatz oder gegebenenfalls auch unmittelbar nachfolgenden Tonsätzen von der Testperson jeweils alle Geräusche als gehört angegeben werden, wird zweckmäßigerweise noch kein (erster) Schätzwert für die Hörschwelle gesetzt.

Unter der Präsentation mehrerer Tonsätze mit "mehrheitlich eine Mehrzahl von Geräuschen" wird hier und im Folgenden insbesondere verstanden, dass grundsätzlich die Tonsätze mehrere abgespielte oder abzuspielende Geräusche enthalten, aber auch vereinzelt Tonsätze mit nur einem Geräusch - im Extremfall sogar ohne Geräusch - vorgesehen sein können. Die Zahl solcher "Einzel"- oder "Leertonsätze" ist dabei gegenüber der Gesamtzahl der Tonsätze gering, liegt bspw. im Bereich von bis zu 20 Prozent.

Unter dem Begriff "Geräusch" wird hier und im Folgenden insbesondere ein geeignetes Test-Geräusch verstanden, das sich von einem üblichen als "reines Sample" aufgenommenen "Alltagsgeräusch" oder "Umgebungsgeräusch" (bspw. Vogelzwitschern, Geräusch eines elektrischen Geräts; das also insbesondere unbekannte Eigenschaften hinsichtlich seiner Einzelbestandteile aufweist) durch vorgegebene und vorzugsweise klar eingegrenzte Eigenschaften unterscheidet. Vorzugsweise handelt es sich bei dem Geräusch um ein synthetisch erzeugtes ("designtes") Geräusch oder auch um ein nach einer Aufnahme bearbeitetes (bspw. durch Filterung unerwünschter Frequenzanteile oder dergleichen) Geräuschsample. In einem "breiten" Sinn stellt das Geräusch also näherungsweise einen Ton insbesondere im musikalischen Sinn dar. Dabei kann dieser Ton (bzw. das Geräusch) bspw. durch einen "reinen" Sinuston, einen modulierten Sinuston, einen Klang (im akustischen Sinn; also bspw. auch einen Akkord), einen sogenannten Wobbel-Ton, ein sogenanntes Schmalbandrauschen oder vergleichbare Schallsignale gebildet sein. Für die vorstehend beschriebenen Geräusche, insbesondere für das Schmalbandrauschen werden vorzugsweise Frequenzbreiten von etwa einer Terz bis zu einer Oktave herangezogen. Zwar können derartige Geräusche (bspw. auch ein Wobbel-Ton oder dergleichen) grundsätzlich eine variierende, bspw. fluktuierende Frequenz aufweisen, da sich diese aber in einem bekannten sowie insbesondere klar abgegrenzten Bereich (bspw. dem für das Schmalbandrauschen angegebenen Frequenzbereich) "bewegt", das Geräusch an sich also gleich bleibt, wird dies hier und im Folgenden vorzugsweise auch als gleichbleibende Eigenschaft verstanden. Im Folgenden wird als äquivalenter Begriff für "Geräusch" für ein einfacheres Verständnis der Begriff "Ton" verwendet - ohne Ausschluss der konkreten Ausgestaltungen des Tons als Sinuston, Schmalbandrauschen etc. Entsprechend wird hier und im Folgenden auch der Begriff "Tonsatz" für einen Satz verwendet, der wie vorstehend bereits beschrieben meist mehrere, im Extremfall aber auch einen oder gar keinen Ton enthalten kann.

Die Töne eines Tonsatzes werden bei der Präsentation dieses Tonsatzes bevorzugt zeitlich nacheinander, gegebenenfalls getrennt durch eine zeitliche Lücke (oder "Pause") dargeboten.

Bevorzugt kommt als Ausgabewandler ein Lautsprecher zum Einsatz. D. h. die Tonsätze, konkret deren Töne werden also insbesondere akustisch dargeboten. Grundsätzlich lässt sich das erfindungsgemäße Verfahren aber auch mit der Ausgabe des jeweiligen Tonsatzes mittels eines Knochenleitungshörers oder eines Cochleaimplantats durchführen.

Vorzugsweise erhält die Testperson keine Information über die in dem jeweiligen Tonsatz enthaltene Zahl von Tönen.

Bei dem "nachfolgenden" Tonsatz, der in Abhängigkeit zu der Antwort auf den (bspw. ersten) "vorausgehenden" Tonsatz verändert wird, handelt es sich optional um den tatsächlich unmittelbar nachfolgenden. Alternativ kann dieser veränderte, nachfolgende Tonsatz aber auch um einen "zwischengeschobenen" (bspw. bereits parallel zu dem vorhergehenden Tonsatz in seinen Tönen ausgewählten) Tonsatz versetzt nachfolgend dargeboten werden. Der Begriff "nachfolgend" beschreibt somit hier und im Folgenden insbesondere den in Abhängigkeit von dem entsprechend vorausgehenden Tonsatz veränderten Tonsatz.

Dadurch, dass die Testperson eine Anzahl von Tönen angeben muss und nicht nur auf die Wahrnehmung eines Tons an sich reagieren braucht, ist das Risiko, dass die Testperson - unbewusst oder auch bewusst zur Verbesserung eines Ergebnisses - eine Falschangabe macht, verringert. Da die Auswahl des entsprechenden nachfolgenden Tonsatzes in Abhängigkeit der abgegebenen Angaben angepasst wird, ist für die Testperson auch ein Testsystem schwieriger abzuschätzen - bspw. im Vergleich zu den nacheinander in ansteigender Lautstärke dargebotenen Tönen im Rahmen eines klassischen Audiogramms. Es sind mithin auf vergleichsweise einfache Weise bessere Testergebnisse zu erwarten.

In einer bevorzugten Verfahrensvariante wird jedem Ton innerhalb des jeweiligen Tonsatzes als eine Eigenschaft jeweils ein unterschiedlicher Lautstärkewert zugewiesen. Das heißt, dass innerhalb eines Tonsatzes unterschiedlich laute Töne dargeboten werden. Gibt in diesem Fall die Testperson eine gegenüber der tatsächlich dargebotenen Zahl an Tönen eine verringerte Zahl an wahrgenommenen Tönen an, kann somit darauf geschlossen werden, dass der Ton mit dem geringsten Lautstärkewert für die Testperson nicht hörbar (also "zu leise") war. Bspw. werden in einem (zu Beginn des Verfahrens gewählten, also "ersten") Tonsatz drei Töne mit Lautstärkewerten von 40, 45 und 50 dB HL (dB "Hearing Level"; diese Maßeinheit ist insbesondere auf die Hörschwelle einer normalhörenden Person bezogen) dargeboten. Gibt die Testperson in diesem Beispiel an zwei Töne gehört zu haben, kann davon ausgegangen werden, dass der Ton bei 40 dB HL nicht hörbar war.

In einer zweckmäßigen Weiterbildung der unmittelbar vorstehend beschriebenen Verfahrensvariante wird zur besonders einfachen, frequenzabhängigen Ermittlung der Hörschwelle (konkret deren frequenzspezifischen Werts) zumindest einer ersten Teilmenge der Tonsätze den Tönen eines jeden Tonsatzes dieser ersten Teilmenge als eine Eigenschaft die gleiche Tonfrequenz (insbesondere im Fall eines Sinustons) oder im Hinblick auf die vorstehend beschriebenen Alternativen, z. B. modulierter Sinuston, Klang, Schmalbandrauschen etc., ein gleicher Frequenzbereich (der bspw. die zu untersuchende Tonfrequenz symmetrisch umgreift) zugewiesen. Anders ausgedrückt werden in mehreren, vorzugsweise aufeinanderfolgend dargebotenen Tonsätze immer die gleichen Töne, nur mit einem unterschiedlichen Lautstärkewert, dargeboten. Dadurch kann zunächst für die aktuelle Tonfrequenz (bzw. den aktuellen Frequenzbereich) auf einfache Weise der Wert der entsprechenden, frequenzspezifischen Hörschwelle bestimmt werden. Vorzugsweise wird daraufhin mittels einer zweiten Teilmenge, insbesondere im Rahmen eines weiteren Durchlaufs mit mehreren Tonsätzen, der Wert der Hörschwelle für eine andere Tonfrequenz (bzw. einen anderen Frequenzbereich) bestimmt. Die im vorhergehend beschriebenen Ausführungsbeispiel dargebotenen drei Töne werden in diesem Fall bspw. (für einen ersten frequenzspezifischen Wert der Hörschwelle) bei (oder im Bereich um) 2000 Hz dargeboten. Ist der frequenzspezifische Wert der Hörschwelle bestimmt, wird vorzugsweise ein vergleichbarer Ablauf für eine weitere Tonfrequenz (bzw. einen weiteren Frequenzbereich) gestartet.

In einer weiteren zweckmäßigen Verfahrensvariante wird insbesondere für den Fall, dass die Tonfrequenz (bzw. der Frequenzbereich) der Töne gleich bleibt, für die Darbietung des nachfolgenden Tonsatzes als Eigenschaft der Lautstärkewert zumindest eines Teils der Töne verändert.

Vorzugsweise wird in einer Weiterbildung zu der vorstehenden Verfahrensvariante der Lautstärkewert zumindest dieses Teils der Töne (insbesondere des nachfolgenden Tonsatzes) in Abhängigkeit von einem Schätzwert, der aus der Anzahl der wahrgenommenen ("gehörten") Töne des vorausgehenden Tonsatzes oder der vorausgehenden Tonsätze gewählt (oder abgeleitet) ist, ausgewählt. Vorzugsweise wird insbesondere als (erster, "grober") Schätzwert für die Hörschwelle ein Wert im Bereich von dem "lautesten" Ton der nicht gehörten Töne bis zu dem nächstlauteren, noch gehörten Ton, bspw. der Lautstärkewert dieses nächstlauteren Tons angenommen. Dieser Lautstärkewert entspricht folglich dem niedrigsten Lautstärkewert der in der als hörbar angegebenen Anzahl von Tönen enthalten ist. Werden also drei Töne (vorzugsweise einheitlicher Tonfrequenz oder des gleichen Frequenzbereichs) - in Fortführung des vorstehend beschriebenen Ausführungsbeispiels bspw. mit Lautstärkewerten von 40, 45 und 50 dB HL - dargeboten und die Testperson gibt an, zwei Töne gehört zu haben, wird der Schätzwert für die Hörschwelle bei dem Ton mit mittlerem Lautstärkewert (hier also bei 45 dB HL) angenommen. Im nachfolgenden (ggf. unmittelbar nachfolgenden) Tonsatz werden dann bspw. drei Töne bei 35, 40 und 45 dB HL dargeboten. Gibt die Testperson hierauf an, nur ("noch") einen Ton gehört zu haben, liegt die Hörschwelle mit hoher Wahrscheinlichkeit bei 45 dB HL. Ebenso können auch (in diesem Tonsatz oder im Rahmen eines weiteren Tonsatzes zusätzlich) die Lautstärkewerte 45, 50 und 55 dB HL dargeboten werden. Gibt die Testperson an, drei Töne gehört zu haben, ist die Wahrscheinlichkeit für die Hörschwelle bei 45 dB HL ebenfalls erhöht.

In einer zweckmäßigen Verfahrensvariante wird die Schrittweite der Lautstärkewerte zwischen den einzelnen Tönen eines Tonsatzes konstant zwischen den einzelnen Tonsätzen gewählt. D. h. in allen dargebotenen Tonsätzen ist der Abstand der einzelnen Lautstärkewerte zueinander gleich.

In einer alternativen Variante wird zur präziseren Eingrenzung des Wertes der Hörschwelle die Schrittweite zwischen den Lautstärkewerten aber ebenfalls verändert. Bspw. werden nach der Darbietung der Lautstärkewerte 40, 45 und 50 dB HL und Angabe, dass zwei Töne gehört wurden, die Lautstärkewerte auf 42, 44 und 45 dB HL gesetzt.

In einer optionalen Verfahrensvariante wird jedem Ton innerhalb des jeweiligen Tonsatzes als eine Eigenschaft jeweils eine unterschiedliche Tonfrequenz (bzw. ein unterschiedlicher Frequenzbereich) zugewiesen. Bspw. wird dabei - zumindest innerhalb eines Tonsatzes oder über eine Teilmenge der Tonsätze hinweg - ein konstanter Lautstärkewert für die Töne gewählt. In diesem Fall wird also die Tonfrequenz (bzw. der Frequenzbereich) bei gleichbleibender Lautstärke variiert, um die jeweiligen frequenzspezifischen Hörschwellenwerte herauszufinden. Alternativ werden sowohl Tonfrequenz (bzw. Frequenzbereich) als auch Lautstärke variiert. In letzterem Fall sind eine kombinatorische Auswertung und/oder ein im Vergleich zu einer konstant bleibenden Eigenschaft komplexerer Versuchsplan erforderlich.

In einer bevorzugten Verfahrensvariante wird als Hörschwelle (konkret als Wert der Hörschwelle) für eine Tonfrequenz der Lautstärkewert festgesetzt, der insbesondere in mehr als 50 Prozent der Tonsätze, aus denen jeweils ein Schätzwert für die Hörschwelle abgeleitet werden kann (in denen also vorzugsweise weder alle Töne noch kein Ton als hörbar angeben wurden), mindestens jedoch in zwei Tonsätzen, als ("noch") hörbar (mithin also als Schätzwert) interpretiert werden konnte, d. h. insbesondere der sich aus den Angaben der Testperson als niedrigster hörbarer Lautstärkewert ableiten ließ. Im Sinne des vorstehend beschriebenen Ausführungsbeispiels wird also der Lautstärkewert 45 dB HL als Wert der Hörschwelle gesetzt, wenn im nachfolgenden Tonsatz, der die Lautstärkewerte 35, 40 und 45 dB HL enthält, seitens der Testperson ein hörbarer Ton angegeben wird.

In einer vorteilhaften Verfahrensvariante wird für einen Teil der Tonsätze ein Ton ausgewählt, der einen Lautstärkewert unterhalb eines zumindest für die Testperson (in jedem Fall) hörbaren Lautstärkewerts aufweist. Bevorzugt wird für diesen Ton der Lautstärkewert dabei aber sogar derart gewählt, dass der Ton unabhängig von der Testperson unhörbar ist, also vorzugsweise unterhalb eines für Personen ohne Hörminderung hörbaren Lautstärkewerts liegt (die Hörschwelle bei normalhörenden Personen liegt üblicherweise bei 0 dB HL, was bei 1000 Hz auch etwa 3 dB SPL entspricht; vorzugsweise wird in dieser Verfahrensvariante "zur Sicherheit" der Lautstärkewert auf -10 dB HL gesetzt). Vorzugsweise wird ein solcher Tonsatz mit einem "normalen" Tonsatz (also einem, der zumindest potentiell von der Testperson hörbare Lautstärkewerte aufweist) abgewechselt. Dadurch wird vorteilhafterweise eine Kontrolle für sogenannte "falsch-positiv" (engl.: "false positive") Antworten ermöglicht. Für den Fall, dass die Testperson insbesondere mehrfach auch bei einem solchen Tonsatz alle Töne als hörbar angibt, wird bspw. eine Warnung oder Fehlermeldung ausgegeben. Diese Variante ist insbesondere hinsichtlich einer Automatisierung des hier und im Folgenden beschriebenen Verfahrens einfach umzusetzen, da lediglich der Lautstärkewert verändert werden braucht.

In einer alternativen (oder optional zusätzlichen) Verfahrensvariante wird zwischen den jeweiligen Tonsätzen auch die Zahl der enthaltenen Töne variiert. "Mehrangaben" von Tönen (also eine höhere Zahl wahrgenommener Töne als dargebotener Töne) ermöglichen dabei ebenfalls einen Rückschluss darauf, dass die Testperson "falsche" Angaben macht. Denkbar ist hier auch, dass für diese Kontrolle vereinzelt auch Tonsätze mit nur einem wiedergegebenen Ton oder im Extremfall auch ohne Ton - oder gegebenenfalls einem zumindest für die Testperson vorzugsweise für eine normalhörende Person unhörbaren Ton - sowie vorzugsweise einer gesamten Tonsatzdauer, die einem Tonsatz mit mehreren Tönen ähnelt, präsentiert werden.

Um die Fehlerunanfälligkeit weiter zu erhöhen, wird in einer zweckmäßigen Verfahrensvariante innerhalb eines Tonsatzes und/oder zwischen den Tonsätzen eine zeitliche Dauer der einzelnen Töne variiert.

Zusätzlich oder alternativ wird die zeitliche Dauer von Lücken ("Pausen") zwischen den einzelnen Tönen variiert. Durch diese zeitliche Variation wird die Wahrscheinlichkeit herabgesetzt, dass die Testperson erfolgreich versucht, die Dauer eines Tonsatzes abzuschätzen und bspw. daraus auf die enthaltene Anzahl von Tönen zu schließen.

Weiter zusätzlich wird auch die Reihenfolge der Töne, insbesondere der Lautstärkewerte (ggf. auch der Tonfrequenzen) innerhalb eines Tonsatzes und vorzugsweise auch zwischen den Tonsätzen variiert. Mit anderen Worten wird nicht nur eine absteigende (d. h. fallende) oder aufsteigende Reihenfolge dargeboten, sondern auch eine gemischte (bspw. Lautstärkewerte in der Reihenfolge 55, 45 und 50 dB HL).

In einer weiteren optionalen Verfahrensvariante werden auch bereits einmal dargebotene Tonsätze insbesondere mit Abstand - d. h. nach mehreren anderen Tonsätzen - wiederholt dargeboten. Dadurch kann die Präzision der Abschätzung des Werts der Hörschwelle weiter gesteigert werden.

Insbesondere für den Fall, dass in einem Tonsatz keine Töne als hörbar angegeben werden, wird im nachfolgenden Tonsatz wenigstens ein Ton mit einem um bspw. 5 dB gegenüber dem höchsten Lautstärkewert des vorhergehenden Tonsatzes erhöhten Lautstärkewert ausgegeben.

Für den Fall, dass alle Töne eines Tonsatzes als hörbar angegeben werden, wird im nächsten Tonsatz vorzugsweise ein Ton ausgegeben, der einen um bspw. 10 dB geringeren Lautstärkewert gegenüber dem geringsten Lautstärkewert des vorhergehenden Tonsatzes enthält.

Für den Fall, dass der höchste (insbesondere für Normalhörende erträgliche oder auch ein maximal ausgebbarer) Lautstärkewert als nicht hörbar angegeben wird (vorzugsweise zweimal), wird das hier und im Folgenden Verfahren vorzugsweise mit einer neuen Teilmenge von Tonsätzen mit Tönen einer anderen Tonfrequenz fortgesetzt.

Zweckmäßigerweise wird vor der eigentlichen Bestimmung der Hörschwelle, d. h. insbesondere vor Auswahl des ersten Tonsatzes, eine Akklimatisierungsphase durchgeführt. Dabei werden - insbesondere für den Fall, dass für alle Töne die gleiche Tonfrequenz oder (im Sinne eines modulierten Tons, Klangs oder eines Schmalbandrauschens) der gleiche Frequenzbereich genutzt werden - der Testperson der für die folgenden Tonsätze, insbesondere für die nachfolgende Teilmenge an Tonsätzen verwendete Ton vorgespielt. Dadurch kann sich die Testperson auf den nachfolgenden Test einstellen.

In einer bevorzugten Verfahrensvariante werden die Tonsätze mittels des Ausgabewandlers, insbesondere eines Lautsprechers eines Hörgeräts, vorzugsweise eines Hörhilfegeräts dargeboten.

In einer zweckmäßigen Verfahrensvariante erfolgen eine Steuerung des Ausgabewandlers, insbesondere des vorstehenden Lautsprechers des Hörgeräts und die Abfrage der Testperson, sowie insbesondere auch die vorstehend beschriebene Veränderung des nachfolgen den Tonsatzes, also zumindest des entsprechenden Teils dessen Töne, durch ein separates Steuergerät. Anders ausgedrückt erfolgt also die Steuerung (oder auch: "Abarbeitung") des vorstehend beschriebenen Verfahrens vorzugsweise durch dieses Steuergerät. Vorzugsweise handelt es sich bei dem Steuergerät um ein mobiles Endgerät, vorzugsweise um ein Smartphone, ein Tablet oder dergleichen mit einer lauffähig installierten Software-Applikation (App), die bei Ausführung auf dem mobilen Endgerät dieses zur bevorzugt automatischen Durchführung des vorstehend beschriebenen Verfahrens ausbildet. Insbesondere umfasst das Steuergerät hierbei einen Mikroprozessor mit einem Datenspeicher, auf dem die vorstehend genannte App gespeichert ist. Der Mikroprozessor arbeitet im bestimmungsgemäßen Betrieb Befehle, die im Programmcode der App enthalten sind, ab und führt so das vorstehend beschriebene Verfahren durch.

Das erfindungsgemäße Computerprogrammprodukt bildet vorzugsweise also die vorstehende App und weist somit Programmcode mit Befehlen auf, die bei der Ausführung des Programmcodes durch einen Prozessor, konkret den vorstehenden Mikroprozessor, diesen veranlassen, das vorstehend beschriebene Verfahren insbesondere automatisch auszuführen.

Das erfindungsgemäße Verfahren zum Einstellen von Hörgeräteparametern eines Hörgeräts einer Testperson, konkret des vorstehend beschriebenen Hörgeräts, vorzugsweise Hörhilfegeräts umfasst das vorstehend beschriebene Verfahren zur Ermittlung der Hörschwelle der Testperson. Darüber hinaus werden nach Ermittlung der Hörschwelle, konkret deren Werts, vorzugsweise des Verlaufs der Hörschwelle über mehrere Tonfrequenzen, Signalverarbeitungsalgorithmen des Hörgeräts auf Basis der Hörschwelle angepasst. Bevorzugt ist auch die Funktionalität zum Einstellen der Hörgeräteparameter in der vorstehend beschriebenen App enthalten.

Das erfindungsgemäße Hörgerätesystem umfasst wenigstens das Hörgerät und ist dazu eingerichtet, das vorstehend beschriebene Verfahren zur Ermittlung der Hörschwelle der Testperson vorzugsweise automatisch, zumindest teilweise in Interaktion mit der Testperson durchzuführen. Vorzugsweise umfasst das Hörgerätesystem dazu das vorstehend beschriebene mobile Endgerät. Das erfindungsgemäße Hörgerätesystem ist also, insbesondere unter Steuerung des Endgeräts, dazu eingerichtet, der Testperson mittels des Ausgabewandlers, insbesondere des Hörgeräts, nacheinander mehrere Tonsätze zu präsentieren, die mehrheitlich eine Mehrzahl von Geräuschen, insbesondere mit jeweils innerhalb eines Tonsatzes gleichbleibenden, vorzugsweise aber untereinander zumindest zum Teil unterschiedlichen, Eigenschaften, enthalten. Das Hörgerätesystem ist außerdem dazu eingerichtet, von der Testperson nach jeder Darbietung eines dieser Tonsätze die Angabe einer wahrgenommenen Anzahl von Geräuschen aus diesem (d. h. insbesondere dem unmittelbar vorausgehend) dargebotenen Tonsatz abzufragen, sowie in Abhängigkeit von der wahrgenommenen Anzahl von Geräuschen wenigstens für die Darbietung des nachfolgenden Tonsatzes eine Eigenschaft zumindest eines Teils der Geräusche (in diesem nachfolgenden Tonsatz) gegenüber dem vorausgehenden Tonsatz zu verändern. Ferner ist das Hörgerätesystem dazu eingerichtet, diesen veränderten, nachfolgenden Tonsatz dann der Testperson darzubieten, und in Abhängigkeit von der jeweiligen wahrgenommenen Anzahl von Tönen in den (insbesondere mindestens zwei, vorzugsweise mehr als zwei) dargebotenen Tonsätzen wenigstens einen Wert - insbesondere einen einzelnen, vorzugsweise frequenzspezifischen Wert und/oder einen Verlauf über mehrere Tonfrequenzen hinweg - der Hörschwelle der Testperson abzuschätzen.

Das Hörgerätesystem sowie die vorstehend genannten erfindungsgemäßen Verfahren (sowie auch das Computerprogrammprodukt bei Ausführung durch den Prozessor) teilen mithin die gleichen Vorteile sowie die sich aus den jeweiligen Beschreibungen ergebenden körperlichen Merkmale.

Die Konjunktion "und/oder" ist hier und im Folgenden insbesondere derart zu verstehen, dass die mittels dieser Konjunktion verknüpften Merkmale sowohl gemeinsam als auch als Alternativen zueinander ausgebildet sein können.

Nachfolgend wird ein Ausführungsbeispiel der Erfindung anhand einer Zeichnung näher erläutert. Darin zeigen:
- Fig. 1: in einer schematischen Darstellung ein Hörgerätesystem, und
- Fig. 2: in einem schematischen Ablaufdiagramm ein von dem Hörgerätesystem durchgeführtes Verfahren.

Einander entsprechende Teile sind in allen Figuren stets mit gleichen Bezugszeichen versehen.

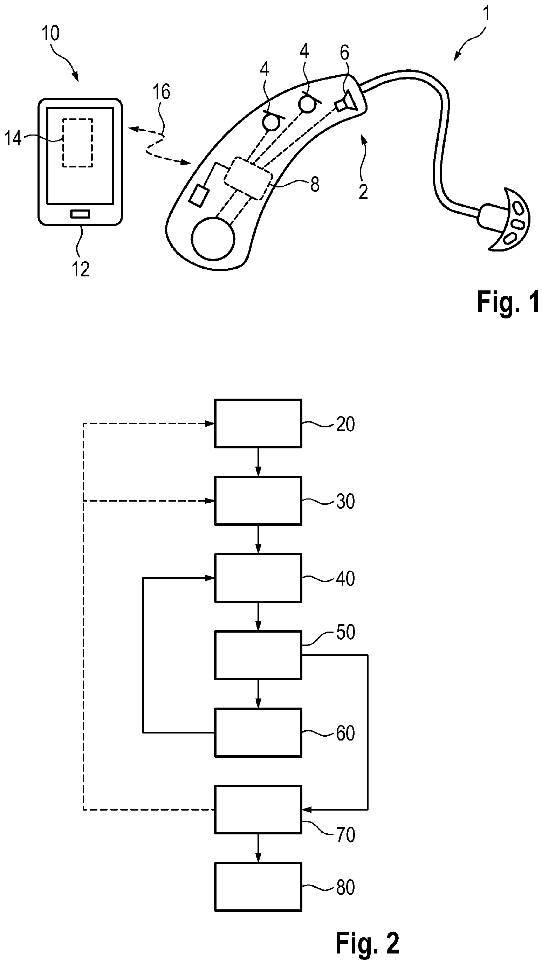

In Fig. 1 ist schematisch ein Hörgerätesystem 1 dargestellt, das zur Versorgung einer Person mit vermindertem Hörvermögen eingerichtet und vorgesehen ist. Das Hörgerätesystem 1 umfasst ein Hörgerät 2, das als Hörhilfegerät, im vorliegenden Ausführungsbeispiel als hinter dem Ohr zu tragendes Hörhilfegerät ausgebildet ist. Das Hörgerät 2 weist dabei als elektronische Komponenten zwei Mikrofone 4, als Ausgabewandler einen Lautsprecher 6 sowie einen Signalprozessor 8 auf. Im bestimmungsgemäßen Betrieb erfassen die Mikrofone 4 Umgebungsgeräusche und geben diese in Form elektrischer Mikrofonsignale an den Signalprozessor 8 weiter. Auf letzterem laufen Signalverarbeitungsalgorithmen ab, deren Parameter eine Anpassung des Hörgeräts 2 an die Art und Schwere der Hörminderung der Person ermöglichen, im bestimmungsgemäßen Betrieb konkret widerspiegeln. Bspw. werden durch die Parameter frequenzspezifische Verstärkungswerte vorgegeben, insbesondere um Mikrofonsignale, deren Lautstärkewert unterhalb eines Werts einer Hörschwelle der Person liegt, anzuheben und somit für die Person (auch als "Hörgeräteträger" bezeichnet) hörbar zu machen. Die verarbeiteten Mikrofonsignale werden anschließend über den Lautsprecher 6 ausgegeben.

Für die Ermittlung der richtigen Parameter wird regelmäßig ein Audiogramm der Person erstellt. Für eine automatisierte Erstellung des Audiogramms weist das Hörgerätesystem 1 ein Steuergerät 10 auf, das eingerichtet ist, ein im Folgenden anhand von Fig. 2 näher beschriebenes Verfahren in Interaktion mit der Person durchzuführen. Das Steuergerät 10 ist konkret durch ein mobiles Endgerät, insbesondere ein Smartphone 12 gebildet, auf dem eine Steuerungssoftware (auch: "Computerprogrammprodukt", im Folgenden aber kurz: "App 14") lauffähig installiert ist. Im bestimmungsgemäßen Betrieb steht das Smartphone 12 mit dem Hörgerät 2 in kabelloser Kommunikationsverbindung 16.

In einem ersten Verfahrensschritt 20 (s. Fig. 2) wird der - in diesem Fall eine Testperson darstellenden - Person ein Ton, im vorliegenden Ausführungsbeispiel ein reiner Sinuston, mit einer spezifischen Tonfrequenz, hier konkret und beispielhaft 2000 Hz vorspielt. Hört die Person diesen (Sinus-) Ton kann der Test zur Ermittlung der frequenzspezifischen Werte der Hörschwelle der Person beginnen. Der Verfahrensschritt 20 stellt eine Akklimatisierungsphase dar.

Alternativ zu dem reinen Sinuston kann auch ein modulierter Sinuston, ein Klang oder ein Schmalbandrauschen mit einem Frequenzbereich von etwa einer Terz bis zu einer Oktave zum Einsatz kommen.

In einem zweiten Verfahrensschritt 30 wird von dem Steuergerät ein erster Tonsatz mit drei Tönen unterschiedlicher Lautstärkewerte, bspw. 60, 50 und 55 dB HL zusammengestellt und der Person über den Lautsprecher 6 des Hörgeräts 2 dargeboten, d. h. vorgespielt. Die einzelnen Töne sind jeweils durch eine Pause voneinander getrennt.

In einem nachfolgenden Verfahrensschritt 40 wird die Person gefragt - konkret wird eine entsprechende Frage auf dem Smartphone 12 angezeigt, optional auch vorgesprochen -, wie viele Töne die Person hören konnte.

In einem weiteren Verfahrensschritt 50 wird die Antwort der Person, die diese auf dem Smartphone 12 eingegeben hat, ausgewertet und für einen weiteren Tonsatz drei neue Töne (mit der gleichen Tonfrequenz) mit teilweise geänderten Lautstärkewerten zusammengestellt. Für den Fall, dass die Person angibt, drei Töne gehört zu haben, werden drei neue Töne zusammengestellt, deren lautester Lautstärkewert 10 dB leiser ist, als der leiseste des vorausgehenden Tonsatzes (im vorliegenden Ausführungsbeispiel also 45, 40 und 35 dB HL). Diese Töne werden im Rahmen eines weiteren Tonsatzes in einem nachfolgenden Verfahrensschritt 60 präsentiert. Wird hingegen keiner der Töne gehört, werden im Verfahrensschritt 60 wiederum drei Töne mit veränderten Lautstärkewerten angeboten, wobei die Lautstärkewerte um wenigstens 5 dB lauter sind als der lauteste Lautstärkewert des vorhergehenden Tonsatzes, hier also 65, 70 und 75 dB HL.

Wird in dem ersten oder einem der nachfolgenden Tonsätze wenigstens ein Ton nicht wahrgenommen, werden die Lautstärkewerte aufsteigend sortiert und die Anzahl nicht gehörter Töne beginnend bei dem niedrigsten Lautstärkewert "gestrichen". Der niedrigste "verbleibende" Lautstärkewert wird dabei als Schätzwert für die Hörschwelle gesetzt. Wird also beispielhaft bei einer Präsentation dreier Töne mit den Lautstärkewerten 50, 55 und 60 dB HL seitens der Person angegeben, dass zwei Töne gehört wurden (was mithin einem nicht wahrgenommenen Ton entspricht), wird also der Schätzwert auf 55 dB HL gesetzt. Für den Fall, dass in dem ersten oder einem unmittelbar nachfolgenden Tonsatz alle enthaltenen Töne oder keine Töne als gehört angegeben werden, wird - anders herum ausgedrückt - zunächst auch kein Schätzwert für die Hörschwelle gesetzt.

Die Pausen (d. h. zeitlichen Lücken) zwischen den einzelnen Tönen sowie die Tonlängen selbst werden dabei innerhalb und zwischen den Tonsätzen variiert.

Nach dem Verfahrensschritt 60 wird auf den Verfahrensschritt 40 zurückgegangen, die Antwort der Person eingeholt und im Verfahrensschritt 50 ausgewertet.

Wird bei wiederholter Präsentation des dem Schätzwert der Hörschwelle entsprechenden Lautstärkewerts dieser ebenfalls wiederholt - für mehr als 50 Prozent bezogen auf die Anzahl der Präsentationen dieses einen Lautstärkewerts - als niedrigster hörbarer Lautstärkewert angegeben, wird zu einem abschließenden Verfahrensschritt 70 übergegangen und dieser Schätzwert als ("endgültiger" oder "bestätigter") Wert der Hörschwelle gesetzt. Anders ausgedrückt wird, wenn für mehr als 50 Prozent aller Präsentationen von Tonsätzen, die wenigstens einen Ton mit gleichem Lautstärkewert enthalten, dieser Lautstärkewert als niedrigster hörbarer Wert ermittelt (und somit als Schätzwert herangezogen) wurde, eben dieser Lautstärkewert im Verfahrensschritt 70 als Wert der Hörschwelle gesetzt. Anschließend wird zum Verfahrensschritt 20 oder 30 zurückgesprungen und mit einer neuen Tonfrequenz das gleiche Verfahren erneut durchgeführt.

Ist für jede Tonfrequenz, bspw. in Schritten von 500, 1000, 2000, 3000 bis 4000 Hz, der Wert der Hörschwelle bestimmt, werden auf dieser Basis in einem weiteren Verfahrensschritt 80 die Parameter neu eingestellt/vorgegeben und an das Hörgerät 2 gesendet.

Der Gegenstand der Erfindung ist nicht auf die vorstehend beschriebenen Ausführungsbeispiele beschränkt. Vielmehr können weitere Ausführungsformen der Erfindung von dem Fachmann aus der vorstehenden Beschreibung abgeleitet werden. Insbesondere können die anhand der verschiedenen Ausführungsbeispiele beschriebenen Einzelmerkmale der Erfindung und deren Ausgestaltungsvarianten auch in anderer Weise miteinander kombiniert werden.

### Bezugszeichenliste

- 1: Hörgerätesystem
- 2: Hörgerät
- 4: Mikrofon
- 6: Lautsprecher
- 8: Signalprozessor
- 10: Steuergerät
- 12: Smartphone
- 14: App
- 16: Kommunikationsverbindung
- 20: Verfahrensschritt
- 30: Verfahrensschritt
- 40: Verfahrensschritt
- 50: Verfahrensschritt
- 60: Verfahrensschritt
- 70: Verfahrensschritt
- 80: Verfahrensschritt

## Patentansprüche

1. Verfahren zur Ermittlung der Hörschwelle einer Testperson, wobei verfahrensgemäß
- einer Testperson mittels eines Ausgabewandlers (6) nacheinander mehrere Tonsätze, die mehrheitlich eine Mehrzahl von Geräuschen, insbesondere mit jeweils innerhalb eines Tonsatzes gleichbleibenden Eigenschaften, enthalten, dargeboten werden,
- von der Testperson nach jeder Darbietung eines dieser Tonsätze die Angabe einer wahrgenommenen Anzahl von Tönen aus diesem dargebotenen Tonsatz abgefragt wird,
- in Abhängigkeit von der wahrgenommenen Anzahl von Tönen für die Darbietung eines nachfolgenden Tonsatzes eine Eigenschaft zumindest eines Teils der Töne gegenüber dem vorausgehenden Tonsatz verändert und der nachfolgende Tonsatz der Testperson dargeboten wird, und
- in Abhängigkeit von der jeweiligen wahrgenommenen Anzahl von Tönen in den dargebotenen Tonsätzen wenigstens ein Wert der Hörschwelle der Testperson abgeschätzt wird.

2. Verfahren nach Anspruch 1,
wobei jedem Geräusch innerhalb des jeweiligen Tonsatzes als eine Eigenschaft jeweils ein unterschiedlicher Lautstärkewert zugewiesen wird.

3. Verfahren nach Anspruch 2,
wobei zur frequenzabhängigen Ermittlung der Hörschwelle zumindest einer ersten Teilmenge der Tonsätze den Geräuschen eines jeden Tonsatzes dieser ersten Teilmenge als eine Eigenschaft die gleiche Tonfrequenz oder ein gleicher Frequenzbereich zugewiesen wird.

4. Verfahren nach Anspruch 2 oder 3,
wobei für die Darbietung des nachfolgenden Tonsatzes als Eigenschaft der Lautstärkewert zumindest eines Teils der Geräusche verändert wird.

5. Verfahren nach Anspruch 4,
wobei der Lautstärkewert zumindest eines Teils der Geräusche in Abhängigkeit von einem aus der Anzahl der wahrgenommenen Geräusche des vorausgehenden Tonsatzes oder der vorausgehenden Tonsätze gewählten Schätzwert der Hörschwelle ausgewählt werden.

6. Verfahren nach einem der Ansprüche 2 bis 5,
wobei die Schrittweite der Lautstärkewerte zwischen den einzelnen Geräuschen eines Tonsatzes konstant zwischen den einzelnen Tonsätzen gewählt wird.

7. Verfahren nach einem der Ansprüche 1 bis 6,
wobei jedem Geräusch innerhalb des jeweiligen Tonsatzes als eine Eigenschaft jeweils eine unterschiedliche Tonfrequenz oder ein unterschiedlicher Frequenzbereich zugewiesen wird.

8. Verfahren nach einem der Ansprüche 1 bis 7,
wobei als Hörschwelle für eine Tonfrequenz der Lautstärkewert festgesetzt wird, der seitens der Testperson für mehr als 50 Prozent der Tonsätze, aus denen sich jeweils ein Schätzwert für die Hörschwelle ableiten lässt, wenigstens jedoch zweimal, als hörbar angegeben wurde.

9. Verfahren nach einem der Ansprüche 2 bis 8,
wobei ein Teil der Tonsätze ein Geräusch mit einem Lautstärkewert enthält, der unterhalb eines zumindest für eine normalhörende Person hörbaren Lautstärkewerts liegt.

10. Verfahren nach einem der Ansprüche 1 bis 9,
wobei innerhalb eines Tonsatzes und/oder zwischen den Tonsätzen eine zeitliche Dauer der einzelnen Geräusche und/oder von Lücken zwischen den einzelnen Geräuschen variiert werden.

11. Verfahren nach einem der Ansprüche 1 bis 10,
wobei die Tonsätze mittels des Ausgabewandlers, insbesondere eines Lautsprechers (6) eines Hörgeräts (2) dargeboten werden.

12. Verfahren nach einem der Ansprüche 1 bis 11,
wobei eine Steuerung des Ausgabewandlers, insbesondere eines Lautsprechers (6), und die Abfrage der Testperson, sowie insbesondere auch die Veränderung des entsprechenden Teils der Töne des nachfolgenden Tonsatzes, durch ein separates Steuergerät (10), insbesondere ein mobiles Endgerät (12) mit einer lauffähig installierten App (14) erfolgt.

13. Computerprogrammprodukt (14), aufweisend Programmcode mit Befehlen, die bei der Ausführung des Programmcodes durch einen Prozessor diesen veranlassen, das Verfahren nach einem der Ansprüche 1 bis 12 auszuführen.

14. Verfahren zum Einstellen von Hörgeräteparametern eines Hörgeräts (2) einer Testperson, wobei verfahrensgemäß die Hörschwelle der Testperson mittels des Verfahrens nach einem der Ansprüche 1 bis 12 ermittelt wird und anschließend Signalverarbeitungsalgorithmen des Hörgeräts (2) auf Basis der Hörschwelle angepasst werden.

15. Hörgerätesystem (1), umfassend wenigstens ein Hörgerät (2) und eingerichtet,
- einer Testperson mittels eines Ausgabewandlers (6) nacheinander mehrere Tonsätze, die mehrheitlich eine Mehrzahl von Geräuschen, insbesondere mit jeweils innerhalb eines Tonsatzes gleichbleibenden Eigenschaften, enthalten, darzubieten,
- von der Testperson nach jeder Darbietung eines dieser Tonsätze die Angabe einer wahrgenommenen Anzahl von Tönen aus diesem dargebotenen Tonsatz abzufragen,
- in Abhängigkeit von der wahrgenommenen Anzahl von Tönen für die Darbietung eines nachfolgenden Tonsatzes eine Eigenschaft zumindest eines Teils der Töne gegenüber dem vorausgehenden Tonsatz zu verändern und den nachfolgenden Tonsatz der Testperson darzubieten, und
- in Abhängigkeit von der jeweiligen wahrgenommenen Anzahl von Tönen in den dargebotenen Tonsätzen wenigstens einen Wert der Hörschwelle der Testperson abzuschätzen.
